# EUROPEAN PATENT APPLICATION

(11) **EP 1 647 280 A2**
(43) Date of publication of application: **19.04.2006**
(21) Application number: 05251785.1
(22) Date of filing: 23.03.2005
(51) Int. Cl.: A61K 38/17, A61P 25/00, A23C 11/06

(54) **Composition for promoting development of nervous system**

(30) Priority: 23.03.2004 JP 2004085589; 23.03.2004 US 555336 P
(71) Applicant: RIKEN, Wako-shi, Saitama 351-0198 (JP); Mikoshiba, Katsuhiko, Tokyo 181-0001 (JP)
(72) Inventor: Mikoshiba, Katsuhiko, Takyo 181-0001 (JP); Hashimoto, Mitsuhiro, Shiki-shi Saitama 353-0007 (JP)
(74) Representative: Richards, William John

(57) **Abstract**

An object of the present invention is to provide a composition, a method, a use thereof, or the like for promoting the development of a mammalian nervous system. The present invention provides a composition for promoting the development of a mammalian nervous system, comprising a fatty acid-binding protein (FABP) fraction as an active ingredient, a method for promoting the development of a mammalian nervous system, comprising administering the FABP fraction to a mammal, a use of the FABP fraction for producing a composition for promoting the development of a nervous system, and the like.

## Description

### FIELD OF THE INVENTION

The present invention relates to a composition for promoting the development of a nervous system, a method for promoting the development of a nervous system, and a use of a specific compound for producing a composition for promoting the development of a nervous system.

### BACKGROUND OF THE INVENTION

A protein involved in intracellular transport of fatty acids is, for example, a fatty acid-binding protein (FABP). FABP is a cytoplasmic protein binding to a long-chain fatty acid and having a molecular weight between 14 kDa and 16 kDa. The protein includes several types having sequence homology, such as a liver type (L-FABP), an intestine type (1-FABP), and a heart-type (H-FABP) according to locations from which the proteins were initially isolated.

In particular, the heart-type fatty acid-binding protein (H-FABP) is a protein with a molecular weight of approximately 15 kDa, which is distributed over a broad range of tissues such as heart, skeletal muscle, stomach, testes, kidney, and mammary glands. The protein is known to be involved in intracellular transport of polyunsaturated fatty acids (linoleic acid, arachidonic acid, and linolenic acid) (non-patent documents 1 to 4). In addition, because the H-FABP content of myocardial cells is high, H-FABP is utilized as a marker for cardiomyopathy such as myocardial infarction (patent documents 1 to 3).

In the meantime, examples of therapeutic agents for affective disorders such as ADHD include atomoxetine (non-patent document 5) as a drug for central stimulation and methylphenidate (non-patent document 6) and amphetamine (non-patent document 7) as psychotropic drugs.

### [Non-patent document 1]

J. H. Veerkamp, R. A. Peeters, R. G. Maatman, Biochim Biophys Acta 1081, 1 (1991).

### [Non-patent document 2]

J. H. Veerkamp, T. H. van Kuppevelt, R. G. Maatman, C. F. Prinsen, Prostaglandins Leukot Essent Fatty Acids 49, 887 (1993).

### [Non-patent document 3]

D. A. Bernlohr, M. A. Simpson, A. V. Hertzel, L. J. Banaszak, Annu Rev Nutr 17, 277 (1997).

### [Non-patent document 4]

T. Hanhoff, C. Lucke, F. Spener, Mol Cell Biochem 239, 45 (2002).

### [Non-patent document 5]

Caballero J, Nahata MC., Clin Ther. 2003 Dec; 25(12): 3065-83.

### [Non-patent document 6]

Aron AR, Dowson JH, Sahakian BJ, Robbins TW. Biol Psychiatry. 2003 Dec 15; 54(12): 1317-29.

### [Non-patent document 7]

Kollins SH., J Clin Psychiatry. 2003; 64 Suppl 11:14-8.

### [Patent document 1]

Specification of US Patent No. 5,690,103 (Groth et al.)

### [Patent document 2]

Specification of US Patent No. 6,099,469 (Armstrong et al.)

### [Patent document 3]

Specification of US Patent No. 6,443,889 (Groth et al.)

### SUMMARY OF THE INVENTION

An object of the present invention is to provide a composition for promoting the development of a nervous system, a method for promoting the development of a nervous system, a use of a specific compound for producing a composition for promoting the development of a nervous system, and the like. Another object of the present invention is to provide a composition for preventing or treating a disease in which the expression of a pheromone receptor is involved, a method for preventing or treating a disease in which the expression of a pheromone receptor is involved, a use of a specific compound for producing a composition for preventing or treating a disease in which the expression of a pheromone receptor is involved, and the like.

In one aspect, the present invention provides a composition comprising a fatty acid-binding protein (FABP) fraction as an active ingredient for promoting mammalian nervous system development. Preferably, the above FABP fraction is a heart-type fatty acid-binding protein (H-FABP) fraction. More preferably, the above FABP fraction is H-FABP. Preferably, the above composition is in a form appropriate for nasal administration, oral administration, or administration by spraying. Furthermore, preferably, the above composition is in the form of spray, powder, liquid, gel, cream, foam, jelly, crystal, liposome, or suspension. Preferably, the above composition is to be administered to an infant and more preferably to a newborn. Furthermore, preferably, the above composition is to be added to a food. In another aspect, the present invention provides a food comprising the above composition, particularly synthetic milk.

In another aspect, the present invention relates to a method for promoting the development of a mammalian nervous system, comprising administering an FABP fraction to a mammal. Preferably, the above FABP fraction is a heart-type fatty acid-binding protein (H-FABP) fraction. More preferably the above FABP fraction is H-FABP. Preferably, the above administration is nasal administration, oral administration, or administration by spraying. Furthermore, preferably, the above FABP fraction is administered in the form of spray, powder, liquid, gel, cream, foam, jelly, crystal, liposome, or suspension. Preferably, administration is performed for an infant, and more preferably for a newborn. Furthermore, preferably, the above FABP fraction is added to a food, and then the fraction contained in the food is administered.

In another aspect, the present invention relates to a use of an FABP fraction for producing a composition for promoting the development of the nervous system. Preferably, the above FABP fraction is a heart-type fatty acid-binding protein (H-FABP) fraction. More preferably the above FABP fraction is H-FABP. Preferably, the above composition for promoting the development of a nervous system is in a form appropriate for nasal administration, oral administration, or administration by spraying. Furthermore, preferably, the above composition for promoting the development of a nervous system is in the form of spray, powder, liquid, gel, cream, foam, jelly, crystal, liposome, or suspension. Preferably the above composition for promoting the development of a nervous system is to be administered to an infant, and more preferably a newborn. Furthermore, preferably, the above composition for promoting the development of a nervous system is added to a food, and then the composition contained in the food is administered.

In another aspect, the present invention provides a composition for promoting mammalian pheromone receptor expression, comprising an FABP fraction as an active ingredient. Preferably, the above pheromone receptor is a V2R type pheromone receptor and more preferably VR1, VR4, or VR1 and VR4 of the V2R type pheromone receptor. Preferably, the above FABP fraction is a heart-type fatty acid-binding protein (H-FABP) fraction and more preferably the above FABP fraction is H-FABP. Preferably, the above composition is in a form appropriate for nasal administration, oral administration, or administration by spraying. Furthermore, preferably the above composition is in the form of spray, powder, liquid, gel, cream, foam, jelly, crystal, liposome, or suspension. Preferably, the above composition is to be administered to an infant and more preferably to a newborn. Furthermore, preferably, the above composition is to be added to a food. In another aspect, the present invention provides a food containing the above composition, particularly synthetic milk.

In another aspect, the present invention provides a method for promoting mammalian pheromone receptor expression, comprising administering an FABP fraction to a mammal. Preferably, the above pheromone receptor is a V2R type pheromone receptor, and more preferably VR1, VR4, or VR1 and VR4 of the V2R type pheromone receptor. Preferably, the above FABP fraction is a heart-type fatty acid-binding protein (H-FABP) fraction, and more preferably the above FABP fraction is H-FABP. Preferably, the above administration is nasal administration, oral administration, or administration by spraying. Furthermore, preferably, the above FABP fraction is administered in the form of spray, powder, liquid, gel, cream, foam, jelly, crystal, liposome, or suspension. Preferably, administration is performed for an infant, and more preferably for a newborn. Furthermore, preferably, the above FABP fraction is added to a food, and then the fraction contained in the food is administered.

In another aspect, the present invention relates to a use of an FABP fraction for producing a composition for promoting the expression of a pheromone receptor. Preferably, the above pheromone receptor is a V2R type pheromone receptor, and more preferably VR1, VR4, or VR1 and VR4 of the V2R type pheromone receptor. Preferably, the above FABP fraction is a heart-type fatty acid-binding protein (H-FABP) fraction, and more preferably, the above FABP fraction is H-FABP. Furthermore, preferably, the above composition is in the form of spray, powder, liquid, gel, cream, foam, jelly, crystal, liposome, or suspension. Preferably, the above composition is in a form appropriate for nasal administration, oral administration, or administration by spraying. Preferably, the above composition is to be administered to an infant, and more preferably to a newborn. Furthermore, preferably, the above composition is added to a food and then the composition contained in the food is administered.

In another aspect, the present invention relates to a composition for preventing or treating a disease in which the expression of a pheromone receptor is involved, comprising an FABP fraction in an effective amount for preventing or treating a disease in which the expression of a pheromone receptor is involved. Preferably, the above pheromone receptor is a V2R type pheromone receptor, and more preferably VR1, VR4, or VR1 and VR4 of the V2R type pheromone receptor. Preferably, the above FABP fraction is a heart-type fatty acid-binding protein (H-FABP) fraction, and more preferably, the above FABP fraction is H-FABP. Furthermore, preferably, the above composition is in the form of spray, powder, liquid, gel, cream, foam, jelly, crystal, liposome, or suspension. Preferably, the above composition is in a form appropriate for nasal administration, oral administration, or administration by spraying. Furthermore, preferably, the above composition is to be added to a food. In another aspect, the present invention provides a food containing the above composition, particularly synthetic milk.

In another aspect, the present invention provides a method for preventing or treating a disease in which the expression of a pheromone receptor is involved, comprising administering an FABP fraction in an effective amount for preventing or treating a disease in which the expression of a pheromone receptor is involved to a subject who requires such prevention or treatment. Preferably, in the above method, the fraction is administered in the form of aerosol by spraying. Preferably, the above pheromone receptor is a V2R type pheromone receptor, and more preferably VR1, VR4, or VR1 and VR4 of the V2R type pheromone receptor. Preferably, the above FABP fraction is a heart-type fatty acid-binding protein (H-FABP) fraction, and more preferably, the above FABP fraction is H-FABP. Furthermore, preferably, the above FABP fraction is administered in the form of spray, powder, liquid, gel, cream, foam, jelly, crystal, liposome, or suspension. Furthermore, preferably, the above FABP fraction is added to a food and then the fraction contained in the food is administered.

In another aspect, the present invention provides a use of an FABP fraction for producing a composition for preventing or treating a disease in which the expression of a pheromone receptor is involved. Preferably, the above pheromone receptor is a V2R type pheromone receptor, and more preferably VR1, VR4, or VR1 and VR4 of the V2R type pheromone receptor. Preferably, the above FABP fraction is a heart-type fatty acid-binding protein (H-FABP) fraction, and more preferably, the above FABP fraction is H-FABP. Furthermore, preferably the above composition is in the form of spray, powder, liquid, gel, cream, foam, jelly, crystal, liposome, or suspension. Preferably, the above composition is in a form appropriate for nasal administration, oral administration, or administration by spraying. Furthermore, preferably, the above composition is added to a food and then the composition contained in the food is administered.

In still another aspect, the present invention provides a composition comprising a breast milk extract for promoting the development of a mammalian nervous system, for promoting mammalian pheromone receptor expression, or for preventing or treating a disease in which the expression of a pheromone receptor is involved. Preferably, the above pheromone receptor is a V2R type pheromone receptor, and more preferably VR1, VR4, or VR1 and VR4 of the V2R type pheromone receptor. Preferably, the above breast milk extract is a fraction containing a protein of less than 100 kDa and more preferably a fraction containing a protein between 10 kDa and 100 kDa. Preferably, the above composition is in a form appropriate for nasal administration, oral administration, or administration by spraying. Furthermore, preferably the above composition is in the form of spray, powder, liquid, gel, cream, foam, jelly, crystal, liposome, or suspension. Preferably the above composition is to be administered to an infant and more preferably a newborn. Furthermore, preferably the above composition is to be added to a food. In another aspect, the present invention provides a food containing the above composition, particularly synthetic milk. Furthermore, the present invention also provides: a use of a breast milk extract for producing the above composition; or a method for promoting the development of a mammalian nervous system, for promoting mammalian pheromone receptor expression, or for preventing or treating a disease in which the expression of a pheromone receptor is involved, which comprises administering a breast milk extract.

### BRIEF DESCRIPTION OF THE DRAWINGS

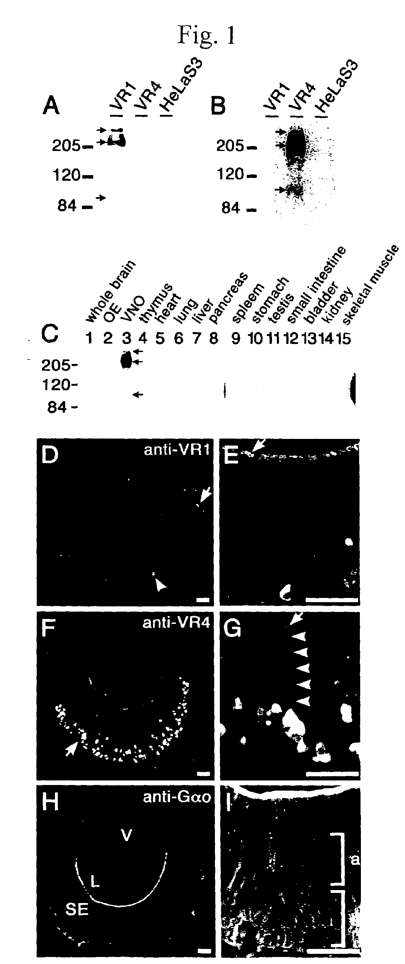

Fig. 1 shows the results of Western blotting analysis on homogenates of HeLaS3 cells infected with expression vectors encoding VR1 and VR4 genes using anti-VR1 (A) and anti-VR4(B) antibodies and the result of Western blotting analysis (C) on homogenates of each tissue excised from adult ICR mice using anti-VR4 antibodies(C). In Fig. 1C, VR4 is expressed only in the vomeronasal organ (lane 3). Both VR1 and VR4 appear to form multimeric homooligomers (indicated with arrows in A to C). In Fig. 1C, 1 denotes whole brain, 2 olfactory epithelium, 3 vomeronasal organ, 4 thymus, 5 heart, 6 lung, 7 liver, 8 pancreas, 9 spleen, 10 stomach, 11 testis, 12 small intestine, 13 bladder, 14 kidney, and 15 skeletal muscle. Fig. 1D to I show the results obtained when the VNO of a 5-week-old male ICR mouse was sectioned transversely with a cryostat (10 µm thickness) and stained with antibodies shown in the figure, wherein "a" denotes apical portion, "b" basal portion, "L" lumen, "SE" sensory epithelium, and "V" blood vessel. Scale bar denotes 100 µm.

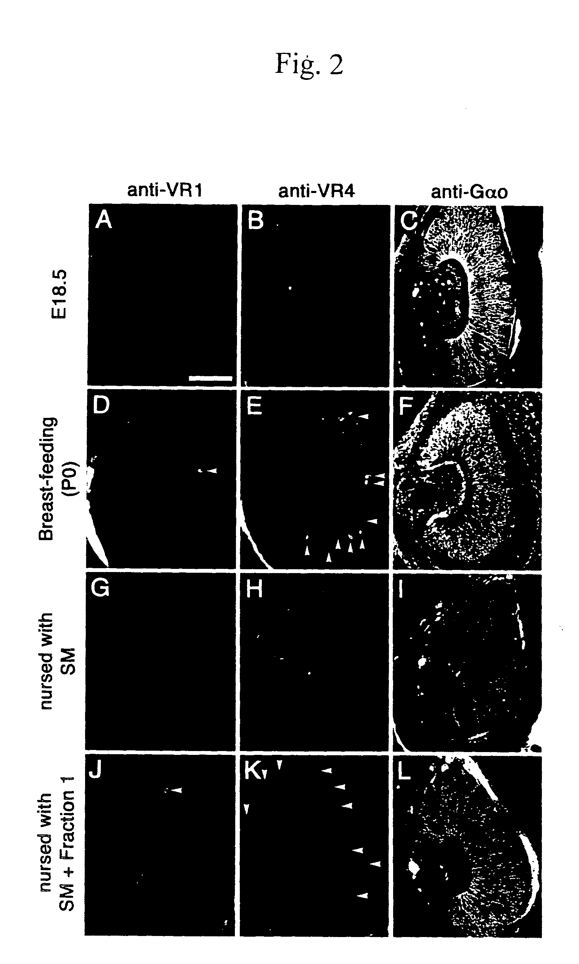

Fig. 2 shows the results of immunostaining of transverse sections of E18.5-day-old embryos and postnatal 0-day-old newborn pups with anti-VR1, anti-VR4, and anti-Gα₀ antibodies, involving the VNOs (A to C) of E18.5 embryos, the VNOs (D to F) of P0 newborn pups after breast-feeding, the VNOs (G to I) of P0 newborn pups hand-fed with skim milk, and the VNOs (J to L) of P0 newborn pups hand-fed with skim milk supplemented with Fraction 1 fractionated from breast milk of dams nursing P 14 pups (see the specification). Arrows indicate the cell bodies of VR1- and VR4-positive VNO neurons. Scale bar denotes 200 µm.

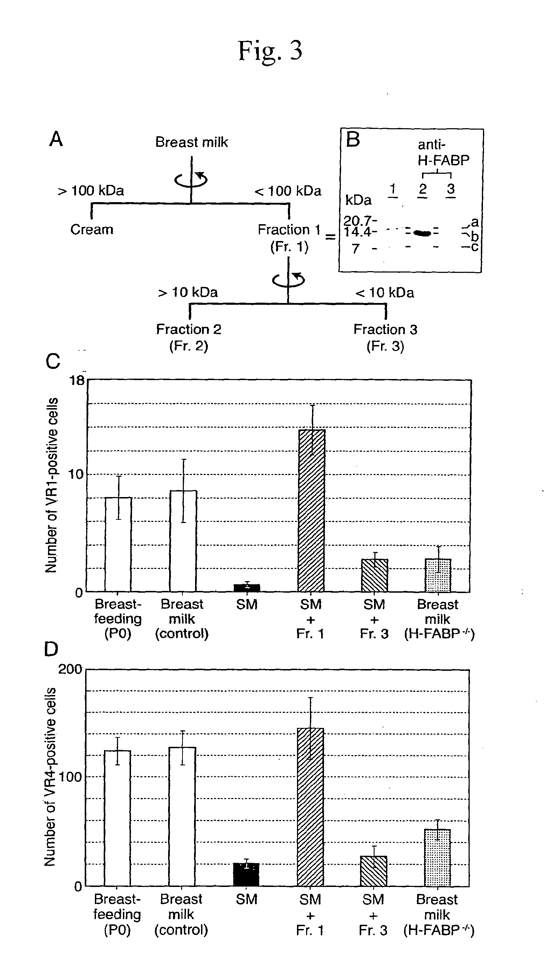

Fig. 3 shows induction of VR1 and VR4 expression by H-FABP in breast milk. The breast milk (P14 breast milk) of dams nursing P14 pups was fractionated based on molecular weight. The fractionation procedure is diagrammed in A. Fraction 1 (Fr. 1) contained α-lactalbumin (band "a": 16.8 kDa), a heart-type fatty acid-binding protein (H-FABP, band "b": 14.8 kDa), and a WDNM1 protein (band "c": 8.2 kDa). Fraction 1 (lane 1 in B) fractionated from P 14 breast milk, fraction 1 (lane 2 in B) obtained from P 14 breast milk of ICR dams, and fraction 1 (lane 3 in B) obtained from P14 breast milk of H-FABP-deficient (H-FABP^{-/-}) dams were subjected to Western blotting using anti-H-FABP antibodies. The fraction 1 obtained from P14 breast milk (lane 3 in B) of H-FABP-deficient (H-FABP^{-/-}) dams lacked H-FABP (band "b"). The number of VR1- and VR4-positive VNO neurons on a series of transversal sections (11 continuous sections at intervals of 100 µm,) of newborn pups (P0) was counted, and diagrammed in graphs C and D, respectively. Each resultant value (mean ± standard deviation) in Fig. 3C is as follows: 8 ± 1.84(n=7) in the case of breast feeding by dams; 8.33 ± 1.09(n=6) in the case of hand-feeding with breast milk; 0.75 ± 0.24(n=9) in the case of hand-feeding with skim milk; 12.33 ± 1.63(n=6) in the case of hand-feeding with skim milk + fraction 1 (SM+Fr.1); 2.5 ± 0.5(n=6) in the case of hand-feeding with skim milk + fraction 3 (SM+Fr. 3); and 2.8 ± 1.1 (n=5) in the case of hand-feeding with breast milk (derived from H-FABP^{-/-} dams). The same in Fig. 3D is as follows: 123.8 ± 12.7(n=6) in the case of breast-feeding by dams; 127.8 ± 10.6(n=11) in the case of hand-feeding with breast milk; 20.6 ± 4.1 (n=8) in the case of hand-feeding with skim milk; 141.7 ± 13.9(n=6) in the case of hand-feeding with skim milk + fraction 1; 28.4 ± 8.5(n=5) in the case of hand-feeding with skim milk + fraction 3 (SM+Fr. 3); and 51.8 ± 9.2(n=5) in the case of hand-feeding with breast milk (derived from H-FABP^{-/-} dams). Error bars in the graphs represent the mean ± standard deviation.

### DETAILED DESCRIPTION OF THE INVENTION

### 1. Definition

In this specification, the term "FABP fraction" means a fatty acid-binding protein (FABP) or a fragment thereof, a variant, a derivative, or an analogue, or a substance that binds to or is associated with FABP. The term "FABP fraction" is intended to also include one of these substances or a mixture of a plurality of these substances. For example, "FABP fraction" includes a functional derivative of FABP, a substance-bound FABP, and a complex of FABP and a substance.

The term "H-FABP fraction" means a heart-type fatty acid-binding protein (H-FABP) or a fragment thereof, a variant, a derivative, or an analogue, or a substance binding to or associated with H-FABP. Furthermore, the term "H-FABP fraction" is intended to also include one of these substances or a mixture of a plurality of these substances. For example, "H-FABP fraction" includes a functional derivative of H-FABP, a substance-bound H-FABP, and a complex of H-FABP and a substance.

The term "FABP" means a protein that is a fatty acid-binding protein (FABP), where such proteins are of a family of intracellular fat-binding proteins with a low molecular weight (14 to 15 kDa). Examples of FABP include a heart-type fatty acid-binding protein (H-FABP), a liver-type fatty acid-binding protein (L-FABP), an adipocyte-type fatty acid-binding protein (A-FABP), a myelin-type fatty acid-binding protein (M-FABP), an intestine-type fatty acid-binding protein (1-FABP), an epithelial fatty acid-binding protein (E-FABP), and a brain-type fatty acid-binding protein (B-FABP) (Veerkamp JH., Proc Nutr Soc. 1995 Mar., 54(1): 23-37; Banaszak L, Winter N, Xu Z, Bernlohr DA, Cowan S, Jones TA., Adv Protein Chem. 1994, 45, 89-151 (all contents are incorporated in this specification by reference in their entirety)). These proteins may be natural, synthetic, or semi-synthetic. Furthermore, when used in this specification, "FABP" indicates not only FABP, which is morphologically and constitutively completely identical to that existing in nature, but also FABP variants, derivatives, and analogues such as an FABP fragment; that is, a molecule of a portion of any FABP amino acid sequence, a protein having an amino acid sequence derived from the amino acid sequence of any FABP by deletion, substitution, and/or addition of one or a plurality of (preferably 1 to 5) amino acid residues, a protein consisting of a sequence that is at least 90% or more identical to any FABP amino acid sequence, and a protein obtained by modification of any FABP with compounds.

The term "H-FABP" means a heart-type fatty acid-binding protein (H-FABP) or its equivalent such as FABP3, a cardiac muscle-type fatty acid-binding protein, a skeletal muscle-type fatty acid-binding protein, or a mammary gland-derived growth inhibitor (MDGI) (Bohmer FD, Kraft R, Otto A, Wernstedt C, Hellman U, Kurtz A, Muller T, Rohde K, Etzold G, Lehmann W, et al., J Biol Chem. 1987 Nov 5, 262(31): 15137-43 (all contents are incorporated in this specification by reference in their entirety)). They may be natural, synthetic, or semi-synthetic.

The term "infant" means children of an age between immediately after birth and complete weaning. In the case of humans, generally the term means children of two and under. Furthermore, the term "newborn" means children under 1 month old.

The term "synthetic milk" means pseudo-breast milk other than breast milk itself. Examples of "synthetic milk" include milk-processing products for oral ingestion and milk preparations.

The term "mammal" means any animal classified as a mammal. Examples of "mammals" include humans, domestic animals, farm animals, and pet animals. Preferably a "mammal" is a human.

The term "subject" includes all persons who require treatment or prevention. Specifically, "subject" includes, in addition to persons who are currently having diseases, persons who may develop diseases in the future, persons who are diagnosed as being likely to develop diseases, and healthy persons.

"Effective amount" or "effective dose" for prevention or treatment means an amount necessary for obtaining a preventive or therapeutic effect, such as an amount necessary for promoting the development of a nervous system, particularly an amount necessary for promoting the expression of a pheromone receptor or an amount necessary for preventing or treating a disease in which the expression of a pheromone receptor is involved.

The term "disease in which the expression of a pheromone receptor is involved" includes all diseases that can be developed due to changes in the expression of pheromone receptors, such as disorders relating to emotion. The term "disorder relating to emotion" is interpreted in a wide sense and includes so-called behavioral disorders such as attention-deficit hyperactivity disorder (ADHD), attention deficit disorder (ADD), anxiety, panic disorder, depression, autism, dysgryphia, Tourette's syndrome, and the like.

### 2. Fatty acid-binding protein (FABP)

Fatty acid-binding proteins are substances functioning as mediators for the transport of cytoplasmic hydrophobic compounds. The proteins are particularly involved in transport and metabolism of fat and are richly present in the cytoplasm. The proteins are known to bind to free fatty acids or CoA derivatives thereof. Among the members of this protein family, a liver type fatty acid-binding protein (L-FABP), an intestine-type fatty acid-binding protein (1-FABP), a heart-type fatty acid-binding protein (H-FABP), an adipocyte-type FABP (A-FABP), an epithelial fatty acid-binding protein (E-FABP), and the like are known.

H-FABP will be explained as follows, but the present invention is not limited thereto. For example, other FABPs can also be produced by a method similar to that employed for H-FABP.

### 3. Heart-type fatty acid-binding protein (H-FABP)

H-FABP is a protein with a molecular weight of approximately 15 kDa distributed in a broad range of tissues such as heart, skeletal muscle, stomach, testis, kidney, and mammary gland. The protein is known to be involved in intracellular transport of polyunsaturated fatty acids (linoleic acid, arachidonic acid, and linolenic acid).

H-FABP has been purified from heart tissue (van Nieuwenhoven FA, Vork MM, Surtel DA, Kleine AH, van der Vusse GJ, Glatz JF., J Chromatogr., 1991 Sep 18, 570(1), 173-9), and can be purified from breast milk by a method known by persons skilled in the art (Grosse R, Boehmer FD, Langen P, Kurtz A, Lehmann W, Mieth M, Wallukat G., Methods Enzymol., 1991, 198, 425-40; Vitaly L. Spitsberg and R. C. Gorewit, Pakistan Journal of Nutrition 1(1): 43-48, 2002 (all contents are incorporated in this specification by reference in their entirety)). For example, H-FABP can be obtained by fractionation by centrifugation or column chromatography based on molecular size, or by affinity purification using antibodies.

Furthermore, H-FABP can be produced in large quantities by causing *Escherichia coli,* yeast, or the like to express H-FABP by gene recombination techniques, and then purifying the product (Peeters RA, Veerkamp JH, Geurts van Kessel A, Kanda T, Ono T., Biochem J. 1991 May 15, 276 (Pt 1), 203-7; Peeters RA, Ena JM, Veerkamp JH., Biochem J. 1991 Sep 1, 278 (Pt 2), 361-4; Schaap FG, Specht B, van der Vusse GJ, Borchers T, Glatz JF., J Chromatogr B Biomed Appl. 1996 Apr 26, 679(1-2), 61-7 (all contents are incorporated in this specification by reference in their entirety)). These methods are already known and can be easily implemented by persons skilled in the art. The nucleotide sequence and the amino acid sequence encoding H-FABP can be easily obtained from, for example, gene sequence databases such as GENBANK. For example, in the DBGET database, bovine H-FABP has been registered under registration number [P10790], human H-FABP under registration number [P05413], mouse H-FABP under registration number [P11404], swine H-FABP under registration number [002772], and rat H-FABP under registration number [P07483]. Furthermore, a nucleotide sequence encoding H-FABP of an animal species other than those of the above animals can be obtained by a known method such as cloning and sequencing from genomic DNA or cDNA samples of a target animal species using the above known sequences as probes.

### 4. Composition

A composition containing the thus obtained FABP fraction can be used as a composition for promoting the development of a mammalian nervous system, for promoting mammalian pheromone receptor expression, or for preventing or treating a disease in which the expression of a pheromone receptor is involved.

### 5. Use as food additive

The composition containing the FABP fraction according to the present invention can be added as a food additive to a food, such as synthetic milk.

It is particularly preferred that the composition or the like according to the present invention be added to synthetic milk, particularly powdered milk, that the resultant be prepared in a manner similar to general preparation of synthetic milk or powdered milk, and that the preparation then be provided. Since H-FABP is a soluble protein, H-FABP is advantageous in that it can be easily mixed into synthetic milk.

Dosage varies depending on target animal species, body weight, and intake. Preferably, the composition in an amount approximately equivalent to that contained in breast milk is mixed. In general, dosage is approximately between 0.1% and 10% and preferably approximately between 0.5% and 2% (W/W) in a food, where such food is preferably synthetic milk, and particularly powdered milk.

The composition according to the present invention is in a form that can be added to a food, such as freeze-dried powder or liquid. They can be added to foods. Moreover, a food to which the composition is added, particularly synthetic milk, is also an object of the present invention. For example, this can be provided in the form of H-FABP-containing powdered milk wherein the composition is in the form of freeze-dried powder has been previously mixed with powdered milk.

Particularly when used in synthetic milk, H-FABP is known to bind to essential fatty acids including linoleic acid, arachidonic acid, and α-linolenic acid. In particular, α-linolenic acid is a raw material for biosynthesis of docosahexaenoic acid (DHA) and is a substance recognized to have usefulness. Since the solubility of fatty acid is low in an aqueous solution, the amount of fatty acid that can be ingested in a processed milk product for oral ingestion is limited. There is another advantage in that by mixing H-FABP, the water-soluble protein, with an essential fatty acid, H-FABP functions as a transport carrier for the essential fatty acid, so as to increase the solubility of the essential fatty acid into an aqueous solution.

### 6. Use as pharmaceutical composition

Furthermore, the thus obtained H-FABP or a fraction or a composition containing H-FABP can also be provided as a pharmaceutical composition. Preferably, the composition contains a pharmaceutically or physiologically acceptable carrier. Moreover, the composition according to the present invention can be used in combination with one or a plurality of other active ingredients, such as a combination with other substances acting on the nervous system or the like.

"Carrier" includes a pharmaceutically acceptable carrier, excipient, or stabilizer and is non-toxic to a subject or a mammal within the range of the amount or concentration thereof to be used. Examples of such carrier include: an organic acid buffer such as phosphate or citrate; an antioxidant such as ascorbic acid; a polypeptide; a protein such as serum albumin, gelatine, or immunoglobulin; a hydrophilic polymer such as polyvinylpyrrolidone; an amino acid such as glycine, glutamine, asparagine, arginine, or lysine; a carbohydrate such as a monosaccharide, e.g., glucose, arabinose, or mannose, a disaccharide, e.g., lactose, saccharose, or maltose, or a polysaccharide, e.g., dextran; a chelating agent such as EDTA; sugar alcohol such as xylitol, mannitol, or sorbitol; salts such as sodium chloride or calcium carbonate; or a nonionic surfactant such as polyethylene glycol, TWEEN (registered trademark), or PLURONIC (registered trademark). The aforementioned may also be used in combination.

The route of administration of the composition according to the present invention may be any method as long as a desired effect can be obtained therewith. The composition can be administered by oral administration, nasal administration, transmucosal administration, or administered by inhalation, such as by spraying. In particular, oral administration, nasal administration, and administration by inhalation are preferable.

The composition according to the present invention can be in a dosage form appropriate for the above routes of administration, such as spray, powder, liquid, gel, cream, foam, jelly, crystal, liposome, or another micelle, a solution, or a suspension. The composition can be administered via liquid agents, dispersing agents, emulsions, suspending agents, bio-adhesive gel or non-bio-adhesive gel, powders, microspheres, lozenge, tablets, chewing gum agents, tablets, capsules, dragee, sustained release preparations, or the like. Administration can take place in combination.

In particular, in the case of nasal administration or administration by inhalation, the composition can be administered via liquid formulations, semi-solid formulations, or dry formulations such as powder formulations using spray, aerosol, an aeration apparatus, an inhalator, or the like. Moreover, the composition can be administered in the form of liquid or a suspending agent sprayed from a spray vessel or in the form of aerosol spray sprayed from a pressurization vessel or the like using an appropriate nebula or the like.

When the composition is administered using aerosol, it is preferable that the formulation be in the form of small particles, such as small particles of 5 microns or less. Such particles can be obtained by a method known in the art. Active ingredients can be administered using an appropriate propellant (for example, a chlorofluorocarbon such as dichlorodifluoromethane, trichlorofluoromethane, or dichlorotetrafluoroethane, or a gas such as carbon dioxide). A surfactant such as lectin may be contained in an aerosol agent. In the case of dry powder, for example, the composition can be administered as a powdered mixture of the composition and a dry base (for example, a starch derivative such as lactose, starch, hydroxypropyl methylcellulose, or polyvinylpyrrolidone (PVP)). These dosage forms can be prepared using techniques known in the art.

The dose of the composition according to the present invention may be any dose, as long as a desired effect can be obtained therewith, such as in the case of an effective amount for treatment or prevention.

Furthermore, H-FABP is a soluble protein and can be conveniently stored by frozen storage, freeze-dry storage, or the like. Hence, the composition is produced in the form of powder, the composition in the form of powder is suspended or dissolved in a water-soluble carrier or in water before administration, and then the resultant can be administered. In addition, the composition may also be in a form that enables preparation and storage in the form of small separated portions for single doses.

### 7. Method and use according to the present invention

The above and following definitions, explanations, and the like relate mainly to the composition according to the present invention. In particular, they relate to a composition for promoting the development of a mammalian nervous system, a composition for promoting mammalian pheromone receptor expression, and a composition for preventing or treating a disease in which the expression of a pheromone receptor is involved. The definitions, explanations, and the like are also similarly applied directly to: the method according to the present invention, particularly the method for promoting the development of a mammalian nervous system, the method for promoting mammalian pheromone receptor expression, and the method for preventing or treating a disease in which the expression of a pheromone receptor is involved; and to a use according to the present invention, particularly a use for producing a composition for promoting the development of a nervous system, a use for producing a composition for promoting the expression of a pheromone receptor, a use for producing a composition for preventing or treating a disease in which the expression of a pheromone receptor is involved, and the like.

Hereinafter, examples of H-FABP, examples of VR1 and VR4 pheromone receptors existing in mouse vomeronasal organs (VNOs), and examples of administration to infants or newborns will be described below. However, the present invention is not limited thereto. In addition, other documents are cited at need as follows, but all contents thereof are incorporated in this specification by reference in their entirety.

### Mammalian vomeronasal organ

The mammalian vomeronasal organ (VNO) is located at the base of the nasal septum and is connected to nasal cavity via a narrow duct. Sensory neurons in the VNO project their axons into the accessory olfactory bulb (AOB). It has been reported in mice that the presence of lesions or genetic inactivation factors in this VNO to AOB pathway may result in abnormal sex discrimination and mating behaviors (E. B. Keverne, Trends in Neurosciences 6, 381 (1983); L. Stowers, T. E. Holy, M. Meister, C. Dulac, G. Koentges, Science 295, 1493 (2002); B. G. Leypold et al., Proc Natl Acad Sci USA 99, 6376 (2002); K. Del Punta et al., Nature 419, 70 (2002)). Consequently, the VNO and the AOB are thought to be essential regions for the detection of pheromones that affect social communication, reproductive behaviors, and endocrine responses. Previous studies also indicated that a dam and her pups communicated with each other through pheromonal signals. It is now generally believed that there is a positive association between dam-pup bonding and breast-feeding (R. H. Porter, J. Winberg, Neurosci Biobehav Rev 23, 439 (1999); B. Schaal et al., Nature 424, 68 (2003)).

Recent studies have reported putative pheromone receptors cloned from the VNO (C. Dulac, R. Axel, Cell 83, 195 (1995); G. Herrada, C. Dulac, Cell 90, 763 (1997); H. Matsunami, L. B. Buck, Cell 90, 775 (1997); N. J. Ryba, R. Tirindelli, Neuron 19, 371 (1997)). These pheromone receptors are members of the G-protein-coupled family of receptors and have been classified into two large groups (R. Tirindelli, C. Mucignat-Caretta, N. J. Ryba, Trends Neurosci 21, 482 (1998); E. B. Keverne, Science 286, 716 (1999); C. Dulac, A. T. Torello, Nat Rev Neurosci 4, 551 (2003)). One type, the V1R type receptors, have short N-terminal extracellular domains. The other type, the V2R type receptors, have characteristically very long N-terminal extracellular domains. While V1Rs respond to volatile pheromone molecules, V2Rs respond to nonvolatile pheromone molecules such as lipocalins (R. Tirindelli, C. Mucignat-Caretta, N. J. Ryba, Trends Neurosci 21, 482 (1998); E. B. Keverne, Science 286, 716 (1999); C. Dulac, A. T. Torello, Nat Rev Neurosci 4, 551 (2003)). These pheromone receptors are thought to be involved in pheromonal communication between a dam and her pups.

### Mammal and breast milk

Mammals need breast feeding by their dams for a certain period after birth. However, in modern society, many non-human mammals are born in an artificial raising environment such as a zoo or a facility for the livestock industry. These mammals are often unable to be breast-fed by their dams due to their environment or the conditions of their dams.

Furthermore, even in the case of humans, many infants are completely unable to be breast-fed, or are insufficiently breast-fed by their mothers due to a variety of reasons. It is assumed that recently the number of such infants is on the increase. Such infants are often raised by hand-feeding with nutrients; that is, synthetic milk. Such synthetic milk contains nutrients that are thought to be necessary for the healthy growth of infants, such as micronutrients, e.g., various minerals, lactose as an energy source, and proteins.

However, recent studies have revealed that breast milk contains various substances to promote infants' biophylaxis, such as an immunoglobulin (IgA) for preventing bacteria or viruses from invading and infecting bodies, bifidus factors for growing intestinal bacteria, lactoferrin, lysozyme, and leucocytes having bactericidal action, and polyamine thought to suppress the onset of allergies. As shown by such findings, breast milk contains not only general nutrients, but also various functional substances. However, there may be many other unidentified breast milk ingredients with unknown functions. Addition of breast-milk-specific functional ingredients to synthetic milk and administration of such synthetic milk to infants are thought to allow the promotion of the growth of infants who are hand-fed with synthetic milk, without impact from insufficient breast feeding or from lack of ability to breast feed.

### Composition and the like according to the present invention

Through the administration of the composition according to the present invention, even in the case of infants who are not breast-fed, induction of the expression of pheromone receptors (particularly, the V2R type receptor) in vomeronasal organs is observed with distribution and levels similar to those observed in breast-fed newborns. Administration of the composition enables even infants who are not breast-fed to carry out appropriate pheromonal reception, so as to be able to promote normal growth.

Reception of pheromonal stimuli is thought to promote induction of the development of a nervous system. Hence, the composition and the like of the present invention can be used as an inducer for pheromone receptor expression, a promoter for the development of a nervous system, or a promoter for the formation of a bond between a mother and her child. Here, "formation of a bond between a mother and her child" means the formation of a psychological bond between a mother and her child, which is induced by neural stimuli. Insufficient formation of this bond may cause symptoms such as a child becoming unable to discriminate his or her mother from other individuals or a child becoming unable to feel love for his or her mother. Furthermore, when such an infant who has performed insufficient bond formation grows, abnormalities may be found in his or her sex-discrimination ability, reproductive behavior, or the like. Furthermore, when such an infant grows and has her child, she may be unable to form a sufficient mother-child bond with her child. Accordingly, if pheromone receptor expression is too late in terms of timing or is insufficient in terms of amount, such a disorder will be passed onto the next generation.

Such pheromonal stimuli are thought to also be involved in emotional development. According to the composition and the like of the present invention, a disease in which a pheromone receptor is involved, particularly, a disease relating to emotion, can be treated or prevented by promoting the development of a pheromone receptor and promoting the following emotional development.

Furthermore, non-human mammals are often kept in artificial environments as pet animals, animals in a zoo, or the like. In such cases, mammals may be unable to be breast-fed by dams. In this case, it is preferable to administer the composition of the present invention in addition to provision of synthetic milk.

Through the administration of H-FABP, pheromone receptor expression is induced similarly to the case of breast feeding, the following development of a nervous system is caused similarly to that in breast-fed animals, and emotional development and mother-child bond formation are promoted, so that the above disorders can be prevented or reduced.

### EFFECT OF THE INVENTION

According to the composition, the method, or the use of the present invention, the development of a mammalian nervous system can be promoted. Furthermore, according to the composition, the method, or the use of the present invention, a disease in which the expression of a pheromone receptor is involved can be prevented or treated.

### EXAMPLES

### Materials and Methods

### Preparation of antibodies against VR1, VR4, and H-FABP

Peptides of the COOH-termini of VR1 (NH₂-CPDSNFIKNHKGKLLY-COOH: SEQ ID NO: 1) and VR4 (NH₂-CPERNSTQKIREKSYF-COOH: SEQ ID NO: 2) of V2R type pheromone receptors of a mouse vomeronasal organ (VNO) and H-FABP (NH₂-CGSVVSTRTYEKEA-COOH: SEQ ID NO: 3) were synthesized. The peptides were then conjugated to keyhole limpet hemocyanin (KLH) according to standard methods. Rabbits were immunized with them as antigens. Specific antibodies against VR1, VR4, and H-FABP were affinity-purified from the anti-sera using affinity columns (Sulfolink column: PIERCE).

### Construction of adenovirus vectors

All of the entire coding sequences of the mouse pheromone receptors, VR1 and VR4, were amplified by the PCR method from the VR1 and VR4 cloned vectors. As VR1 primers, a forward primer: 
5'-CCGGATCCTCGCCACCATGAAGCAGCTCTGCGC-3' (SEQ ID NO: 4) and a
reverse primer: 5'-GCCTCGAGTCAATAAAGCAATTTACCTTTGTGG-3' (SEQ
ID NO: 5) were used. As VR4 primers, a forward primer:
5'-CCCTCGAGCGCCACCATGTTCATTTTCATGGGAGTC-3' (SEQ ID NO: 6)
 and a reverse primer: 5'-GCTCTAGATCAAAAATATGATTTTTCTCTGATCTTTTGGG-3' (SEQ ID NO: 7) were used. The amplified products were cloned into pBluescript II(KS-)(Stratagene). The sequences were confirmed by sequencing. The *BamH I-Xho* I fragment of VR1 cDNA and the *Xho I-Xba* I fragment of VR4 cDNA were then inserted into adenovirus expression vectors. The vectors were then introduced into HeLaS3 cells using Lipofectamine 2000 (Invitrogen).

### Nursing newborn pups by hand-feeding

ICR (Slc: ICR; NipponSLC, Japan) and H-FABP-deficient mice (B. Binas, H. Danneberg, J. McWhir, L. Mullins, A. J. Clark, FASEB J 13, 805 (1999)) were housed in a controlled environment under a regulated 12-hour light:dark cycle. The day upon which a vaginal plug was detected was considered to be day E0.5 (0.5-day old embryo). Day E19.5 was designated as postnatal day 0 (P0). Mouse breast milk was collected from ICR and H-FABP-deficient dams breast-feeding pups at P14 (P14 breast milk). The milk was subjected to molecular weight fractionation by ultracentrifugation using Ultrafree 0.5 [100 kDa nominal molecular weight limits (NMWL): Millipore], thereby obtaining 100 kDa or more breast milk fractions and 100 kDa or less breast milk fractions (fraction 1). Fraction 1 was separated by Ultrafree 0.5 (10 kDa NMWL: Millipore), thereby obtaining fraction 2 and fraction 3. Each fraction was stored at -80°C.

ICR mouse newborn pups were delivered by Caesasrian section on day E18.5, immediately isolated from dams, and placed on pure cotton. Newborn pups were maintained at 37°C and swabbed until they began breathing. Immediately after confirmation of their ability to breathe through their noses, the newborn pups were hand-fed with P14 breast milk obtained from ICR dams, 20%(w/v) skim milk (SM: Wako), 20% (w/v) SM supplemented with breast milk fraction 1 at a 1:10 ratio, or P14 breast milk obtained from H-FABP-deficient dams. Specifically, drops of each nutrient were added to the pups' mouths, and it was visually confirmed that each pup had approximately 50 to 100 µl of the nutrient introduced into its stomach. The pups were kept under control until the next day (P0). The hand-feeding with each nutrient was performed on a litter of pups.

### SDS-PAGE and Western blotting

Tissues of adult ICR mice and HeLaS3 cells transfected with VR1 or VR4 expression adenovirus vectors were homogenized in 20 mM Tris-HCl buffer (pH 7.5) containing 250 mM sucrose, 10 mM EDTA (pH 8.0), 1 mM β-mercaptoethanol, and 0.2 mM phenylmethylsulphonyl fluoride on ice. The protein concentration of each homogenate sample was measured using a protein assay kit (Bio-Rad). Each homogenate (10 µg protein/lane) was subjected to 10% SDS-PAGE. The filtrate of P 14 breast milk (breast milk of dams having P14 pups) or the biotinylated filtrate of P14 breast milk was subjected to 16.5% tricine-SDS-PAGE. Detection was carried out by Coomassie staining.

Gel was transferred to PVDF membranes (Immobilon: Millipore). Membranes were blocked overnight with PBS(-) containing 1% skim milk (Becton Dickinson) and 0.1% Tween 20 (Nacalai Tesque, Inc.) at 4°C. The membranes were caused to react with antibodies against VR1 (1 µg/ml) or VR4 (0.5 µg/ml) for 2 hours. Following several washes, immunoreactivity was detected using secondary antibodies (ECL kit: Amersham).

### Immunostaining

Two days after the transfection with the VR1 or VR4 adenovirus expression vector, HeLaS3 cells were fixed in 4% (w/v) paraformaldehyde (PFA) in 0.1 M phosphate buffer (pH 7.4), and subsequently washed with PBS(-). Day E18.5 embryos and P0 newborn pups were anesthetized and fixed by intracardiac perfusion with 4% PFA. The VNOs of 5-week-old ICR mice were also excised and then the VNOs were fixed overnight at 4°C. Samples were isolated as described in literature (M. Hashimoto et al., Hum Gene Ther 7, 149 (1996)), and then transverse-sectioned (10 µm thickness at 100 µm intervals) using a cryostat.

HeLaS3 cells and sections were washed with PBS(-), subjected to blocking with PBS(-) containing 1% skim milk and 0.1% Triton X-100 (Sigma) for 1 hour, and then caused to react with VR1 (2 µg/ml) or VR4 (1 µg/ml) antibodies. The neighboring sections were caused to react with anti-Gα₀ IgG (1:200; MBL). Positive immunoreactivity was detected with a rhodamine-labeled anti-rabbit IgG (1:200; CAPPEL).

To examine the development of pheromone receptors, immunostaining was carried out using antibodies that specifically recognize VR1 and VR4 of V2R type pheromone receptors, respectively. Specific antibodies against the C-terminal intracellular domains of VR1 and VR4 were affinity-purified from the immunized rabbit antisera and analyzed by immunological analyses (data not shown), Western blotting, and immunohistochemical analysis.

Homogenate samples of HeLaS3 cells that had been transfected with vectors expressing VR1 and VR4 were subjected to Western blotting (Fig. 1A and B). The HeLaS3 cells do not express endogenous VR1 and VR4 (see "HeLaS3" in Fig. 1A and B). The anti-VR1 and anti-VR4 antibodies showed specific reaction only in the HeLaS3 cells expressing VR1 ("VR1" in Fig. 1A) and VR4 ("VR4" in Fig. 1B). The anti-VR1 and anti-VR4 antibodies did not show any cross-reaction with VR4 and VR1, respectively.

To determine the *in vivo* distribution of V2R type pheromone receptors, Western analysis was carried out on various tissue homogenates derived from adult ICR mice (Fig. 1C). Anti-VR4 immunoreactivity was only detectable in the VNO (lane 3 in Fig. 1C). In the case of the sample, three immunoreactive bands were observed (indicated with arrows in Fig. 1A to C). The major bands of the lowest molecular weights agreed with the expected sizes (approximately 100 kDa) of the VR1 and VR4 monomers. The molecular weights of the two upper bands (detected on the higher molecular weight side than these major bands) correspond to molecular weights twofold and fourfold greater than the molecular weights of the monomers, respectively.

The bands corresponding to a putative receptor dimer were the most strongly detected. These results indicate that V2R type pheromone receptors (VR1 and VR4) exist in multimeric conformations. Furthermore, sections of adult mouse VNO were immunostained with anti-VR1 (Fig. 1D and E), anti-VR4 (Fig. 1F and G), and anti-Gα₀ (Fig. 1H and I) antibodies. Anti-VR1 and anti-VR4 immunoreactivity was observed at the tips of the dendrites (indicated with arrows in Fig. 1E and G), dendrites (indicated with arrows in Fig. 1G), and cell bodies of the VNO. The cell bodies of VR1 (or VR4)-positive VNO neurons were found in the basal half of the VNO sensory epithelium and they were also positive for Gα₀ (Fig. 1H and I). Anti-VR1 and anti-VR4 immunoreactivity was not observed within the olfactory bulb or within the accessory olfactory bulb (AOB) of adult mice (data not shown). The above anti-VR1 and anti-VR4 immunoreactivity became undetectable upon absorption of these antibodies by the immunizing peptide epitope (data not shown). It was proved that immunoreaction is due to a specific reaction.

### Induction of expression of VR1 and VR4 after birth

Using these specific antibodies, the distribution of VR1 and VR4 pheromone receptors within the VNOs of embryos and newborn pups was examined. VR1 and VR4 were undetectable in embryonic VNOs at embryonic day (E) 15.5, E17.5 (data not shown), and E18.5 (Fig. 2A and B), but Gα₀ (Fig. 2C) was expressed, suggesting maturation of these neurons. For VNO neurons of newborn pups (postnatal day 0: P0) fed with dams' breast milk, the expression of both VR1 (indicated with an arrow in Fig. 2D) and VR4 (indicated with arrows in Fig. 2E) was detected. Thus, it was revealed that the expression of VR1 and VR4 pheromone receptors is rapidly induced within 1 day after birth. Recent studies have showed that synaptic connections between VNO sensory axons and the dendrites of AOB neurons are rapidly formed after birth (L. F. Horowitz, J. P. Montmayeur, Y. Echelard, L. B. Buck, Proc Natl Acad Sci U.S.A. 96, 3194 (1999)) and that Trp2 protein (a channel located downstream of pheromone receptor signaling) is detected after birth (E. R. Liman, D. P. Corey, C. Dulac, Proc Natl Acad Sci U.S.A. 96, 5791 (1999)). It has also been shown that the expression of M10 and β2-microglobulin (they are also involved in pheromonal responses) are also induced after birth (J. Loconto et al., Cell 112, 607 (2003)). Consequently, embryonic VNO neurons are unable to accept pheromonal stimuli and pheromonal reception is developed immediately after birth.

### Induction of VR1 and VR4 expression by breast milk

The identity of the factor that rapidly induces upregulation of VR1 and VR4 expression remains unknown. However, any of the following 6 factors may serve as stimuli inducing the upregulation of VR1 and VR4 expression: parturition, first breath, dam's licking stimulation, dam's saliva, dam's odors, and breast-feeding. To examine this question, ICR mouse pups were delivered by Caesarean section on day E18.5 and then put on pure cotton, kept at 37°C, and stimulated to breathe by swabbing in a manner similar to a dam's licking. After first breath, the newborn pups were nursed with either skim milk or mouse breast milk (P 14 breast milk) obtained from a dam nursing P 14 pups, while being isolated from dams.

On the next day (postnatal day 0 (P0)), each newborn pup was fixed by intracardiac perfusion with 4% paraformaldehyde and sectioned transversely on a cryostat. Sections were stained with anti-VR1 and anti-VR4 antibodies. In newborn pups nursed with skim milk, anti-VR1 and anti-VR4 immunoreactivity (Fig. 2G and H) of VNO neurons was significantly reduced compared with those of the VNOs of newborn pups (Fig. 2D and E) breast-fed by their dam. In the meantime, anti-Gα₀ immunoreactivity (Fig. 2F and I) was the same for both of the two. The VNOs of newborn pups hand-fed with P 14 breast milk (data not shown) exhibited a similar distribution of VR1 and VR4 expression as that in the VNOs of newborn pups breast-fed by dams (Fig. 2D and E). To clarify the differential effects of skim milk and P14 breast milk, the number of VR1-positive and VR4-positive VNO neurons in a series of transversal sections (100 µm thickness; 11 sections) was determined (Fig. 3C and D). In comparison with pups nursed with breast milk ("breast milk" in Fig. 3C and D), the number of VR1- and VR4-positive VNO neurons of pups nursed with skim milk ("SM" in Fig. 3C and D) was significantly reduced to 9% (P<0.001) and 16.1 % (P<0.001) of pups breast-fed by dams, respectively. During the hand-feeding procedure, pups experienced parturition, first breath, and swabbing that simulated the dams' licking stimulation. However, they were not exposed to dams' saliva or odors. Consequently, these results suggest that parturition, first breath, and dams' licking stimulation do not induce VR1 and VR4 expression, and that dams' saliva and odors are not necessary to induce VR1 and VR4 expression. Breast milk is the most likely potential factor inducing VR1 and VR4 expression.

### Inducing factor in breast milk: H-FABP

To identify the factor in breast milk that induces VR1 and VR4 expression, P14 breast milk was fractionated by ultracentrifugation using a molecular weight fractionation method into two fractions, a fraction (fraction 1) consisting of proteins of less than 100 kDa and a fraction consisting of proteins of 100 kDa or more. Fraction 1 was liquid containing lactose, water, ions, water-soluble molecules, and whey proteins, and the other fraction was in the form of cream. Next, newborn pups derived from litters delivered by Caesarean section were divided into 4 groups: a group of pups that was breast-fed by dams, a group of pups that was hand-fed with P14 breast milk, a group of pups that was hand-fed with skim milk, and a group of pups that was hand-fed with skim milk supplemented with fraction 1 (SM+Fr.1). While VR1 and VR4 expression was induced in the group nursed with skim milk supplemented with fraction 1, VR1 and VR4 expression was not induced in the group nursed with only skim milk ("SM" in Fig. 2G and H and Fig. 3C and D). The distribution of VR1 and VR4 expression in the group fed with SM+Fr. 1 ("SM+Fr.1" in Fig. 2J and K and Fig. 3C and D) was similar to that in the group breast-fed by dams ("breast-fed by dams" in Fig. 2D and E and Fig. 3C and D). The number of VR1- and VR4-positive VNO neurons was slightly increased in the group nursed with SM+Fr. 1 ("SM+Fr. 1" in Fig. 3C and D). The results indicate that the activity inducing VR1 and VR4 expression resides in fraction 1. Fraction 1 was further fractionated into proteins (fraction 2) of 10 kDa or more and proteins (fraction 3) of less than 10 kDa containing other water-soluble molecules (Fig. 3A). Newborn pups delivered by Caesarean section were hand-fed with skim milk (SM+Fr. 3) supplemented with fraction 3. The number of VR1- and VR4-positive VNO neurons in pups hand-fed with SM+Fr. 3 ("SM+Fr. 3" in Fig. 3C and D) was significantly reduced to approximately 20% (VR1 and P<0.001; VR4 and P<0.001) of the levels seen in pups nursed with SM+Fr. 1 ("SM+Fr. 1" in Fig. 3C and D). Fraction 2 retained the same level of VR1 and VR4 induction activity as seen for SM+Fr. 1 (data not shown). The results suggest that the molecule inducing VR1 and VR4 expression has a molecular weight between 10 kDa and 100 kDa.

To examine components of the proteins fractionated in fraction 1, fraction 1 was separated by SDS-PAGE (lane 1 in Fig. 3B). Fraction 1 contained three bands (bands a, b, and c in Fig. 3B). Proteins were extracted from each band, and then the proteins contained in each band were identified by liquid chromatography tandem mass spectrometry (data not shown). Band (a) was identified as mouse α-lactalbumin. Band (b) was a mouse heart-type fatty acid-binding protein (H-FABP). Band (c) was a mouse WDNM1 protein. Of these three types of protein, we considered that H-FABP was a factor for inducing pheromone receptor expression. Hence, newborn pups (delivered by Caesarean section) were hand-fed with P14 breast milk derived from H-FABP-deficient dams, and then the effect of H-FABP contained in the breast milk was confirmed.

When pups were hand-fed with P 14 breast milk derived from H-FABP-deficient dams, the number of VR1- and VR4-positive VNO neurons was significantly reduced (VR1, P<0.01; VR4, P<0.005) ("breast milk (H-FABP^{-/-})" in Fig. 3C and D) from the levels observed in control animals ("breast milk" in Fig. 3C and D). Fraction 1 of the P14 breast milk of the H-FABP-deficient dams lacked H-FABP (band "b" in lane 3 of Fig. 3B), but retained α-lactalbumin (band "a") and WDNM1 (band "c") expression (data not shown). These results indicate that H-FABP acts to rapidly induce VR1 and VR4 expression by breast-feeding. Thus, it was shown by this experiment that H-FABP in breast milk rapidly induces the expression of V2R type pheromone receptors after birth. Breast-feeding may facilitate pheromone reception of pups by inducing V2R type pheromone receptors, thus enriching pheromonal communication between a dam and her pups. Hence the above results suggest that a breast milk ingredient (e.g., H-FABP) affects the development of neural tissue (e.g., induction of pheromone receptor expression in VNOs). Breast-feeding may confer benefits on neural development of pups.

The present invention can be implemented in other various forms without departing from the spirit or principles of the present invention. Hence, the above examples are given merely for illustrative purposes in every respect, and should not be construed in a limited way. The scope of the present invention is defined by the scope of the claims, and is never restrained by description given in the specification. Furthermore, all modifications and variations within the scope of equivalents of the scope of the claims are within the scope of the present invention.

## Claims

1. A composition for promoting the development of a mammalian nervous system, comprising a fatty acid-binding protein (FABP) fraction as an active ingredient.

2. The composition of claim 1, wherein the FABP fraction is a heart-type fatty acid-binding protein (H-FABP) fraction.

3. The composition of claim 1, wherein the FABP fraction is H-FABP.

4. The composition of claim 1, which is in the form of spray, powder, liquid, gel, cream, foam, jelly, crystal, liposome, or suspension.

5. The composition of claim 1, which is to be administered to an infant.

6. The composition of claim 1, which is to be added to a food.

7. Synthetic milk, comprising the composition of claim 6.

8. A method for promoting the development of a mammalian nervous system, comprising administering an FABP fraction to a mammal.

9. A use of an FABP fraction for producing a composition for promoting the development of a nervous system.

10. A composition for promoting mammalian pheromone receptor expression, comprising an FABP fraction as an active ingredient.

11. The composition of claim 10, wherein the pheromone receptor is a V2R type pheromone receptor.

12. The composition of claim 10, wherein the pheromone receptor is VR1, VR4, or VR1 and VR4 of the V2R type pheromone receptor.

13. The composition of claim 10, wherein the FABP fraction is an H-FABP fraction.

14. The composition of claim 10, wherein the FABP fraction is H-FABP.

15. The composition of claim 10, which is in the form of spray, powder, liquid, gel, cream, foam, jelly, crystal, liposome, or suspension.

16. The composition of claim 10, which is to be administered to an infant.

17. The composition of claim 10, which is to be added to a food.

18. Synthetic milk, comprising the composition of claim 17.

19. A method for promoting mammalian pheromone receptor expression, comprising administering an FABP fraction to a mammal.

20. The method of claim 19, wherein the pheromone receptor is a V2R type pheromone receptor.

21. The method of claim 19, wherein the pheromone receptor is VR1, VR4, or VR1 and VR4 of the V2R type pheromone receptor.

22. A use of an FABP fraction for producing a composition for promoting the expression of a pheromone receptor.

23. A composition for preventing or treating a disease in which the expression of a pheromone receptor is involved, comprising an FABP fraction in an effective amount for preventing or treating a disease in which the expression of a pheromone receptor is involved.

24. The composition of claim 23, wherein the FABP fraction is an H-FABP fraction.

25. The composition of claim 23, wherein the FABP fraction is H-FABP.

26. The composition of claim 23, wherein the pheromone receptor is a V2R type pheromone receptor.

27. The composition of claim 23, wherein the pheromone receptor is VR1, VR4, or VR1 and VR4 of the V2R type pheromone receptor.

28. The composition of claim 23, which is in the form of spray, powder, liquid, gel, cream, foam, jelly, crystal, liposome, or suspension.

29. A method for preventing or treating a disease in which the expression of a pheromone receptor is involved, comprising administering an FABP fraction in an effective amount for preventing or treating a disease in which the expression of a pheromone receptor is involved to a subject who requires such prevention and treatment.

30. The method of claim 29, comprising administering the fraction in the form of aerosol by spraying.

31. A use of an FABP fraction for producing a composition for preventing or treating a disease in which the expression of a pheromone receptor is involved.


SEQUENCE LISTING

<110> RIKEN
<110> Katsuhiko Mikoshiba

<120> A composition for enhancing the development of neuronal system

<130> PH-2112EP

<140>
<141>

<150> JP2004-085589
<151> 2004-03-23

<150> US60/555336
<151> 2004-03-23

<160> 7

<170> PatentIn Ver. 2.1

<210> 1
<211> 16
<212> PRT
<213> Artificial Sequence

<220>
<223> Description of Artificial Sequence: C-terminal amino acid sequence of VR1

<400> 1
Cys Pro Asp Ser Asn Phe Ile Lys Asn His Lys Gly Lys Leu Leu Tyr
 1               5                  10                  15

<210> 2
<211> 16
<212> PRT
<213> Artificial Sequence

<220>
<223> Description of Artificial Sequence: Description of Artificial Sequence:
C-terminal amino acid sequence of VR4

<400> 2
Cys Pro Glu Arg Asn Ser Thr Gln Lys Ile Arg Glu Lys Ser Tyr Phe

1                   5                   10                  15

<210> 3
<211> 14
<212> PRT
<213> Artificial Sequence

<220>
<223> Description of Artificial Sequence: Description of Artificial Sequence:
C-terminal amino acid sequence of H-FABP

<400> 3
Cys Gly Ser Val Val Ser Thr Arg Thr Tyr Glu Lys Glu Ala
  1               5                  10

<210> 4
<211> 33
<212> DNA
<213> Artificial Sequence

<220>
<223> Description of Artificial Sequence:primer

<400> 4
ccggatcctc gccaccatga agcagctctg cgc                                33

<210> 5
<211> 33
<212> DNA
<213> Artificial Sequence

<220>
<223> Description of Artificial Sequence:primer

<400> 5
gcctcgagtc aataaagcaa tttacctttg tgg                                33

<210> 6
<211> 36
<212> DNA
<213> Artificial Sequence

<220>
<223> Description of Artificial Sequence:primer

<400> 6
ccctcgagcg ccaccatgtt cattttcatg ggagtc                                36

<210> 7
<211> 40
<212> DNA
<213> Artificial Sequence

<220>
<223> Description of Artificial Sequence:primer

<400> 7
gctctagatc aaaaatatga tttttctctg atcttttggg                            40